# EUROPÄISCHE PATENTSCHRIFT

(11) **EP 2 785 278 B1**
(45) Veröffentlichungstag und Bekanntmachung des Hinweises auf die Patenterteilung: **22.03.2017**
(21) Anmeldenummer: 12797771.8
(22) Anmeldetag: 15.11.2012
(51) Int. Cl.: A61F 2/08, A61B 17/04

(54) **IMPLANTIERBARES SYSTEM MIT ELASTISCHEN KOMPONENTEN**
IMPLANTABLE SYSTEM HAVING ELASTIC COMPONENTS
SYSTÈME IMPLANTABLE À COMPOSANTS ÉLASTIQUES

(30) Priorität: 30.11.2011 DE 102011087404
(43) Veröffentlichungstag der Anmeldung: 08.10.2014
(60) Teilanmeldung: 14194661.6
(73) Patentinhaber: Mathys AG Bettlach, 2544 Bettlach (CH)
(72) Erfinder: DELFOSSE, Daniel, CH-3303 Jegenstorf (CH); DE CESARIS, Alessandro, CH-4712 Laupersdorf (CH); DRANSFELD, Clemens, CH-8280 Kreuzlingen (CH); RYTKA, Christian, 79713 Bad Säckingen (DE)
(74) Vertreter: Körfer, Thomas
(86) Internationale Anmeldenummer: PCT/EP2012/072676
(87) Internationale Veröffentlichungsnummer: WO 2013/079327

(56) Entgegenhaltungen:
- EP-A1- 0 238 263
- WO-A1-97/30649
- WO-A1-97/36557
- WO-A1-2010/124760
- GB-A- 2 454 251
- US-A- 4 301 551

## Beschreibung

Die Erfindung bezieht sich auf ein implantierbares System mit elastischen Komponenten zur kontrollierten Beanspruchung eines rekonstruierten oder renaturierten Bandes eines menschlichen oder tierischen Körpers.

Das menschliche Kniegelenk wird durch das vordere Kreuzband und das hintere Kreuzband im Innenraum des Kniegelenks stabilisiert. Bei einem Verdrehtrauma des Kniegelenks werden diese beiden Bänder, insbesondere das vordere Kreuzband, überlastet und es kommt infolge dessen zu einer Ruptur bzw. zu einem Abreißen des Bandes. Führen konservative Therapieversuche oder Versuche, das vordere Kreuzband zu nähen, nicht zu einer Stabilisierung des verletzten Kniegelenks, so wird oft das vordere Kreuzband entfernt und die Kniegelenkstabilität durch ein Transplantat aus Sehnenmaterial oder einem synthetischen Band wiederhergestellt. Dabei wird das beschädigte natürliche Band endgültig aus dem Kniegelenk entfernt. Das künstliche Ersatzband kann jedoch dessen Funktion nur unzureichend übernehmen. Insbesondere geht die Sensibilität komplett verloren, was zu einer Überlastung und somit zu einem höheren Verschleiß der künstlichen Bandprothese führt.

Diese Tatsache und die Selbstheilungstendenz eines jeden Bandes des menschlichen Körpers wird bei einem in der WO 2010/124760 A1 beschriebenen System zur kontrollierten Beanspruchung eines rekonstruierten oder renaturierten Bandes ausgenutzt. Während der Ausheilungsphase wird dabei ein Verbindungselement durch ein Verankerungs- bzw. Halteelement an den beiden zu verbindenden Knochen befestigt, das für eine Entlastung des natürlichen Bandes sorgt und somit das natürliche Zusammenwachsen des Bandes ermöglicht. Das Verankerungselement weist eine Dämpfungseinrichtung auf, um eine gleichmäßige Zugspannung im System zu gewährleisten und das Beugen des behandelten Knies zu ermöglichen. Nach dem Ausheilen des natürlichen Bandes hat das implantierbare System seine Aufgabe erfüllt und wird nicht länger benötigt. Die WO 2010/124760 A1 offenbart, dass eine oder mehrere Komponenten des Systems aus einem bioresorbierbaren Material bestehen und sich selbst auflösen.

Insbesondere Komponenten eines solchen implantierbaren Systems, die aus metallischem Material bestehen, werden von Patienten als störend und schmerzverursachend beschrieben und müssen somit nach der Heilung des Bandes operativ oder arthroskopisch entfernt werden. Ein solcher Eingriff birgt Risiken und Unannehmlichkeiten für den Patienten und verursacht zusätzliche Kosten.

GB-A-2 454 251 offenbart ein System für eine kontrollierte Beanspruchung eines künstlichen Bandes. Es wird die Möglichkeit erwähnt, ein Elastomerelement zu verwenden, um ein möglichst natürliches Verhalten eines künstlichen Bandapparats nachzubilden. Die Festigkeitscharakteristik eines Spannungs- bzw. Federelementes kann linear oder nicht-linear ausgebildet werden.

Es ist somit die Aufgabe der vorliegenden Erfindung, möglichst viele Komponenten des implantierbaren Systems aus einem Material aufzubauen, das gut verträglich für den menschlichen Körper ist und kaum zu Irritationen oder Schmerzen beim Verbleib im Patienten führt. Das implantierbare System soll dabei eine Elastizität ähnlich dem Elastizitätsverlauf des natürlichen Bandes aufweisen.

Die Aufgabe wird durch das erfindungsgemäße System gemäß Anspruch 1 gelöst. In den Unteransprüchen sind vorteilhafte Weiterbildungen des erfindungsgemäßen Systems angegeben.

Das erfindungsgemäße System für eine kontrollierte Beanspruchung eines rekonstruierten oder renaturierten Bandes eines menschlichen oder tierischen Körpers umfasst ein Verankerungselement zur Implantation in einem ersten Knochen, mindestens ein Verbindungselement sowie ein Haltelement, welches das zumindest eine Verbindungselement an einem zweiten Knochen fixiert. Erfindungsgemäß ist ein Elastomerkörper in dem Verankerungselement und/oder in dem Verbindungselement angeordnet und weist durch das Zusammenwirken von Elastomerkörper mit dem Verankerungselement bzw. dem Verbindungselement eine definierte elastische Wirkung auf.

Durch die Auswahl und Zusammensetzung der Materialien des Elastomerkörpers können Elastizitätsmodule in einem weiten Bereich realisiert werden. Durch das Zusammenwirken des Elastomerkörpers mit dem Verankerungselement sowie dem Verbindungselement kann durch günstige Material- und Strukturkombinationen ein Elastizitätsverlauf des Systems ähnlich dem eines natürlichen Bandes erreicht werden. Des Weiteren sind viele Elastomere biokompatibel, sodass sie keine schädliche Wirkung auf menschliches oder tierisches Gewebe haben. Durch ihre Verformbarkeit wirken sie weit weniger irritierend und können somit auch nach der Ausheilung des Bandes im Körper verbleiben.

Vorteilhafterweise ist der Elastomerkörper aus Polyethylen und/oder Polyester und/oder Polyurethan und/oder Silikon. Die genannten Materialien haben bei einem Einsatz im menschlichen Körper biokompatible Eigenschaften und besitzen zudem ein günstiges elastisches Verhalten.

Bei einem Zusammenwirken des Elastomerkörpers mit einem Verbindungselement ist es von Vorteil, wenn der Elastomerkörper einen kleineren Elastizitätsmodul aufweist als das Verbindungselement. Dadurch dominiert bei niedriger Zugspannung am Verbindungselement das Elastizitätsmodul des Elastomers und bei starker Zugspannung dominiert der signifikant größere Elastizitätsmodul des Materials des Verbindungselements. Somit entspricht die Dehnungskennlinie des Verbindungselements in Wechselwirkung mit dem Elastomerkörper der Dehnungskennlinie eines natürlichen Bandes.

Besonders vorteilhaft ist es, wenn das Verbindungselement aus einer Mehrzahl von ersten Einzelfasern geflochten und/oder gestrickt gesponnen und/oder verdrillt und/oder gewoben ist und jede erste Einzelfaser im Bezug zur Längsachse des Verbindungselements einen Winkel τ zwischen 5° und 85°einschließen. Dadurch erlangt das Verbindungselement die Möglichkeit, sich in axiale Richtung zu verlängern und eine elastische Wirkung zu erreichen, auch wenn die Einzelfasern selbst aus einem nicht oder nur sehr gering elastischen Material sind.

Vorteilhafterweise ist das Verbindungselement schlauchförmig ausgebildet und der Elastomerkörper ist in einem Abschnitt des axialen Hohlraums des schlauchförmigen Verbindungselements angeordnet. Der Verlauf der Elastizitätskurve, auch Dehnungskennlinie genannte, einer solchen Kombination weist einen progressiven Verlauf auf, da durch die große Verformung der Druckquerschnitt und die örtliche Spannungskonzentration, auch Formzahl genannt, ansteigen. Zusätzlich wird der progressive Verlauf der Dehnungskennlinie durch den Winkel beeinflusst, mit dem die Einzelfasern des Verbindungselements im Bezug zur Längsachse desselben gewoben, geflochten, gesponnen, verdrillt und/oder gestrickt sind. Bei einer zunehmenden Zugbelastung dominiert zuerst der Elastizitätsverlauf des Elastomerkörpers, der durch den Druck des schlauchförmigen Verbindungselements in radialer Richtung auf den Elastomerkörper bestimmt ist. Gleichzeitig werden die Einzelfasern des Verbindungselements aus einem Winkel τ zur Längsachse ohne Zugbelastung zu einem Winkel von nahezu 0° zur Längsachse bei starker Zugbelastung ausgerichtet. Die Kombination aus Verbindungselement und Elastomerkörper kann sich nicht mehr weiter ausdehnen und die Elastizität nimmt stark ab.

Eine ähnliche Dehnungskennlinie weist ein Verbindungselement auf, bei dem das Elastomer als eine Mehrzahl von zweiten Einzelfasern ausgebildet ist und mit einer Mehrzahl von ersten Einzelfasern zu einem Verbindungselement gesponnen und/oder gestrickt und/oder gewoben und/oder gedrillt und/oder geflochten ist Es ist von Vorteil, wenn die Einzelfasern ein Polymer, insbesondere Polyethylen und/oder Polyester, enthalten und das Verbindungselement aus einer Mehrzahl von Einzelfasern, insbesondere aus Polyethylen und/oder Polyester, gebildet wird. Die genannten Polymere weisen einen relativ hohen Elastizitätsmodul auf, sodass nach einer Streckung des Fasergeflechts die Elastizität der Kombination stark erhöht wird. Außerdem sind diese Polymere biokompatibel und daher für die Implantation in den Körper geeignet.

Vorteilhaft ist es ebenfalls, wenn ein Elastomerkörper als Dämpfungsvorrichtung im Innenraum eines Außenkörpers des Verankerungselements zwischen einer Anlagefläche des Außenkörpers und einer Hülse angeordnet ist. Das Verbindungselement ist dabei an der Hülse fixiert und durch eine Öffnung in der Anlagefläche des Außenkörpers aus dem Verankerungselement herausgeführt. Der Elastomerkörper übernimmt somit die Aufgabe eines Dämpfungselements und kann z.B. eine Anordnung aus metallischen Federn ersetzen.

Dabei ist es von Vorteil, wenn die Hülse beweglich im Außenkörper gelagert ist. Durch die bewegliche Hülse wird bei Zugspannung am Verbindungselement die Hülse selbst gegen den Elastomerkörper, der selbst durch Anlage an das Verankerungselement ortsfest gelagerten, gedrückt und führt zu einer Kompression des Elastomerkörpers.

Dabei ist insbesondere vorteilhaft, wenn das Verbindungselement in einer axialen Aussparung im Elastomerkörper angeordnet ist. Beim Zusammendrücken des Elastomerkörpers dehnt sich der Elastomerkörper auch in axiale Richtung aus und klemmt dabei das Verbindungselement ein. Dies stellt eine weitere starke Zunahme des Elastizitätsmoduls der Kombination aus Verankerungselement und Elastomerkörper dar.

Von Vorteil ist es ebenfalls, wenn das Verbindungselement schraubenförmig bzw. spiralförmig um den Umfang des Elastomerkörpers geführt ist. Bei einer Zugbelastung des Verbindungselements wirkt eine radial nach innen gerichtete Kraft auf den Umfang des Elastomerkörpers und drückt diesen zusammen. Die Steifigkeit des Elastomerkörpers bewirkt somit eine gedämpfte Verlängerung des implantierbaren Systems. Auch hier nimmt die Kompressibilität des Elastomerkörpers mit zunehmender Einschnürung ab und der Elastizitsmodul steigt bei zunehmender Kompression an. Ist die Hülse beweglich im Verbindungselement angeordnet, wirkt zusätzlich eine Kompression in axiale Richtung.

Vorteilhaft ist es ebenfalls, wenn die Hülse unbeweglich am Außenkörper fixiert ist, insbesondere wenn die Federkurve des Systems nicht durch eine zusätzliche axiale Kompression beeinflusst werden soll.

Es ist weiterhin vorteilhaft, wenn der Elastomerkörper umfängliche Nuten aufweist, die nicht parallel zur Längsachse des Elastomerkörpers verlaufen und das Verbindungselement in diesen Nuten geführt ist. Diese Nuten verhindern ein Abrutschen des Verbindungselements und gewährleisten einen kontrollierten Druckansatzpunkt auf den Elastomerkörper. Des Weiteren vereinfacht eine solche Anordnung das Zusammensetzen des Verankerungselements bzw. das Einfädeln des Fadens durch das Verankerungselement.

Vorteilhaft ist ebenfalls ein Elastomerkörper, der als eine Mehrzahl von zungenförmigen Ausbuchtungen ausgeformt ist, wobei die Ausbuchtungen im Wechsel an gegenüberliegenden Seiten von der Innenseite des Außenkörpers des Verankerungselements nach innen gerichtet und radial überlappend angebracht sind. Das Verbindungselement ist dabei im Zwischenraum zwischen den Ausbuchtungen geführt. Bei einer Zugspannung auf das Verbindungselement werden die zungenförmigen Ausbuchtungen des Elastomerkörpers in Richtung der Zugkraft verformt. Bei einer zunehmenden Verformung der Ausbuchtungen verringert sich der Abstand zwischen den Ausbuchtungen und der Faden wird zwischen den Ausbuchtungen eingeklemmt. Dies bewirkt eine starke Zunahme des Elastizitätsmoduls.

Von Vorteil ist es, wenn der Elastomerkörper eine zylindrische Form aufweist. Der Elastizitätsmodul sowie die Kompressibilität des Elastomerkörpers hängen stark von der Form des Elastomerkörpers ab. Die zylindrische Form ermöglicht eine gleichmäßig radial nach innen wirkende Kraftübertragung. Die zylindrische Form erlaubt aber auch eine gute Kraftübertragung in axialer Richtung.

Die Erfindung wird im Folgenden anhand von Ausführungsbeispielen und in Bezug auf die Zeichnungen näher erläutert. Es zeigen:
- Fig. 1: ein erfindungsgemäßes System implantiert in ein Kniegelenk in schematischer Darstellung;
- Fig. 2: eine schematische Darstellung einer Dehnungskennlinie eines weichen biologischen Gewebes im Vergleich zu anderen Materialien;
- Fig. 3A: ein erstes erfindungsgemäßes Ausführungsbeispiele eines Verbindungselements mit Elastomerkörper in schematischer Darstellung;
- Fig. 3B: ein zweites erfindungsgemäßes Ausführungsbeispiele eines Verbindungselements mit Elastomerkörper in schematischer Darstellung;
- Fig. 4: eine Schnittdarstllung durch ein erstes Ausführungsbeispiel eines erfindungsgemäßen Verankerungselements mit einem Elastomerkörper;
- Fig. 5: einen Schnitt durch ein zweites Ausführungsbeispiel eines erfindungsgemäßen Verankerungselements mit Elastomerkörper und
- Fig. 6: einen Schnitt durch ein drittes Ausführungsbeispiel eines erfindungsgemäßen Verankerungselements mit zungenförmigem Elastormerkörper.

Einander entsprechende Teile sind in allen Figuren mit den gleichen Bezugszeichen versehen.

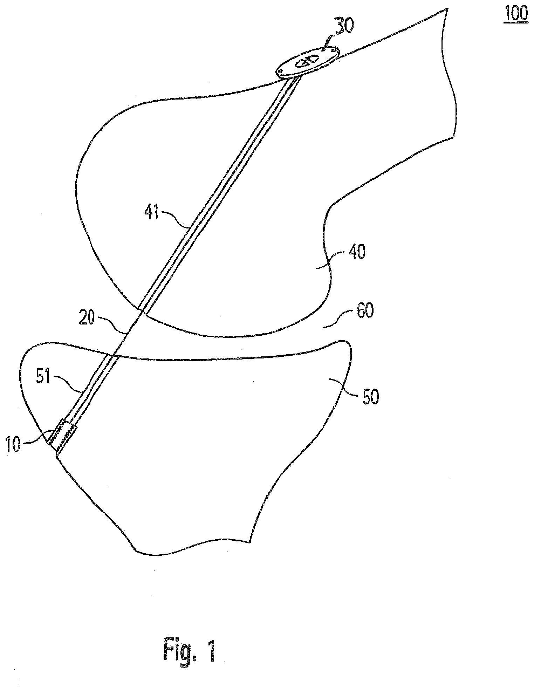

Fig. 1 zeigt das erfindungsgemäße System 100 eingesetzt in ein gebeugtes menschliches Kniegelenk. Das Verankerungselement 10 ist im Ausführungsbeispiel ventral im proximalen Bereich des Tibiaknochens 50 eingeschraubt, an den sich ein erster Knochentunnel 51 anschließt, der zum Gelenkinnenraum 60 führt. Durch das angrenzende distale Ende des Femurknochens 40 ist ein schmaler zweiter Knochentunnel 41 gebohrt. Dort ist das Verbindungselement 20 an einem Halteelement 30 fixiert. Das Halteelement stützt sich dabei an der Außenfläche des Femurknochens 40 ab. Das Verbindungselement führt durch den zweiten Knochentunnel 41 über den Gelenkinnenraum 60 und dem ersten Knochentunnel 51 zum Verbindungselement und wird von diesem fixiert.

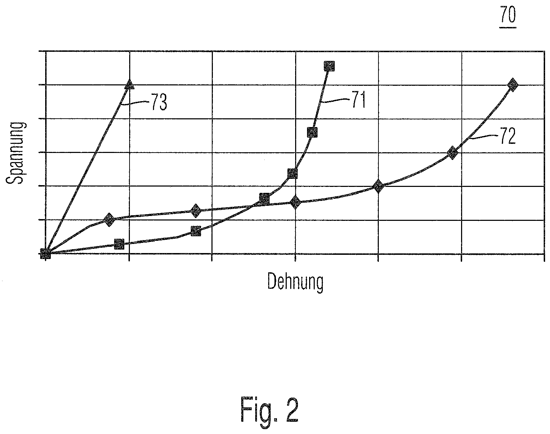

Fig. 2 zeigt ein Diagramm mit Dehnungskennlinien unterschiedlicher Materialen, in dem die anliegende Spannung bei einer bestimmten Dehnung aufgetragen ist. Kennlinie 71 stellt die typische Dehnungskennlinie von weichem biologischem Gewebe, wie z.B. einem menschlichen Band, dar. Die Federkennlinie 72 wurde an einem gummielastischem Polymer, die Linie 73 an Vergütungsstahl aufgenommen. Wie aus dem Diagramm hervorgeht, weisen Elastomere eine progressive Dehnungskennlinie ähnlich der von weichem biologischem Gewebe auf. Der Elastizitätsmodul bzw. die Dämpfungskonstante, die durch die Steigung der Kurve angegeben sind, weist bei geringer Dehnung einen ähnlich flacheren Verlauf auf. Die Progression setzt aber erst bei größerer Dehnung und mit verminderter Stärke ein. Metallische Elemente wie z.B. Vergütungsstahl weisen dagegen ein Elastizitätsmodul auf, das der Federkennlinie im hinteren progressiven Bereich nahekommt.

Es hat sich überraschenderweise gezeigt, dass der Elastizitätsmodul eines Elastomerkörpers, der in ein schlauchförmiges Verbindungselement integriert ist oder sonst mit dem Verbindungselement zusammenwirkt, der Dehnungskennlinie eines natürlichen Bandes angenähert werden kann.

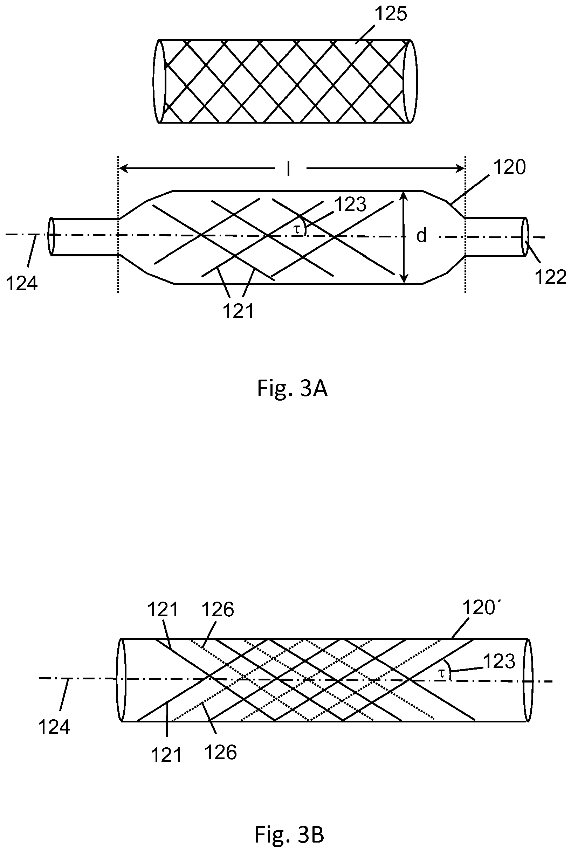

Fig. 3A stellt ein schlauchförmiges Verbindungselement 110 dar, in dessen axialen Hohlraum 122 ein Elastomerkörper 125, der zur Verdeutlichung nochmals separat oberhalb des Verbindungselements dargestellt ist, angeordnet ist. In einem beispielsweise 120 mm langen schlauchförmigen Verbindungselement 120 ist ein zylinderförmiger Elastomerkörper 125 mit einer Länge 1 von bevorzugt 20 mm - 100mm, beispielsweise 60 mm und mit einem Durchmesser von bevorzugt 0,5 mm bis 10 mm, beispielsweise 2 mm, integriert. Das Verbindungselement 120 besteht aus einer Mehrzahl von ersten Einzelfasern 121, die zu einem schlauchförmigen Verbindungselement 120 gestrickt, geflochten, gewebt, verdrillt, und/oder gedreht sind.

Die ersten Einzelfasern 121 bestehen dabei bevorzugt aus Polyethylen und/oder Polyester. Das Verbindungselement 120 kann sowohl aus ersten Einzelfasern 121 eines einzigen Materials, aber auch aus ersten Einzelfasern 121 aus unterschiedlichen Materialien hergestellt werden. Dabei sind diese Einzelfasern 121 unter einem Winkel τ 123 zur Längsachse 124 des Verbindungselements 120 ausgerichtet. Durch das Zusammenwirken des Elastomerkörpers 125 mit dem schlauchförmigen Verbindungselements 120 zeigt das Verbindungselement 120 bei geringer Zugkraft eine große Ausdehnung, d.h. ein niedriges Elastizitätsmodul, das stark zunimmt, sobald der Winkel τ 123 zwischen den Einzelfasern 121 und der Längsachse 124 sich verringert bzw. gegen Null geht. Der Winkel τ liegt bevorzugt zwischen 5° und 85°, besonders bevorzugt zwischen 35° und 55°.

Fig. 3B zeigt ein Verbindungselement 120' bei dem der Elastomerkörper aus einer Mehrzahl von zweiten Einzelfasern 126 ausgebildet ist. Diese sind mit den ersten Einzelfasern 121 zu einem Verbindungselement 121', bevorzugt ohne axialen Hohlraum, zusammengesponnen. Das Verbindungselement 121' kann jedoch auch geflochten, gestrickt, verdrillt, gesponnen und/oder gewoben sein und einen axialen Hohlraum aufweisen.

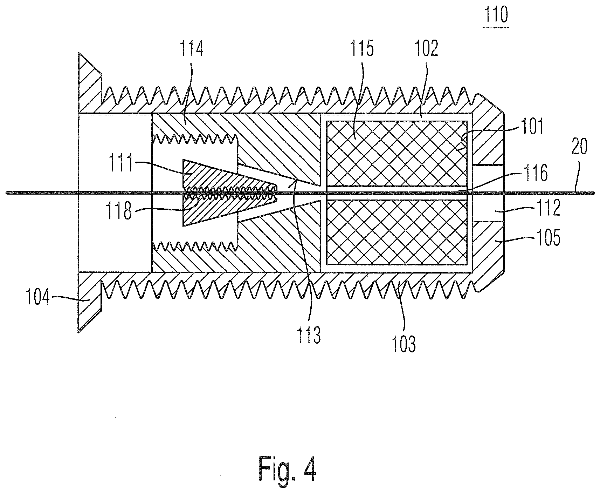

Die Figuren 4-6 zeigen jeweils ein Verankerungselement 110, 110', 110", in dem ein Elastomerkörper 115, 117, 119 als Dämpfungselement wirkt. Ein Verankerungselement 110, 110', 110" weist einen beispielsweise zylinderförmigen Außenkörper 103 auf, der topfförmig ausgebildet ist und an dessen Boden 105 eine Öffnung 112 eingebracht ist. Der zylindrische Außenkörper 103 umfasst z.B. ein Außengewinde, mit dem das Verankerungselement in den ersten Knochen 50, siehe Fig. 1, eingeschraubt wird. An der offenen Stirnseite 104 des Außenkörpers 103 ist eine Hülse 114 eingeführt, die verschiebbar im Innenraum des Außenkörpers gelagert ist. Die Hülse 114 weist ein Befestigungselement 111 auf, das beispielsweise aus zwei Kegelsegmenten besteht. Zwischen den Innenflächen 118 des Befestigungselements 111 ist das Verbindungselement 20 verrutschsicher fixiert. Das Befestigungselement 111 selbst wird bei Zugbelastung gegen die gegengleich geformten Anlageflächen 113 der Hülse 114 gepresst und ist somit fest in der Hülse 114 fixiert.

In Fig. 4 befindet sich ein Elastomerkörper 115 zwischen der Anlagefläche 101 des Außenkörpers 103 und einer parallel gegenüber liegenden Anlagefläche der Hülse 114. Der Elastomerkörper 115 füllt den Innenraum 102, insbesondere in radialer Richtung, nicht komplett aus. Der Elastomerkörper 115 weist eine koaxiale Aussparung 116 auf, durch die das Verbindungselement 20 hindurchgeführt ist und durch die Öffnung 112 des Verankerungselement in Richtung Knochentunnel 51 verlässt.

Bei einer einsetzenden Zugbelastung füllt der Elastomerkörper 115 das freie Volumen im Innenraum 102 kontinuierlich aus, was sich in einem flachen Verlauf der Dehnungskennlinie widerspiegelt. Sobald der Elastomerkörper 115 das gesamte Volumen nahezu ausfüllt, kommt es aufgrund der Inkompressibilität des Elastomers zu einem exponentiellen Anstieg der Kurve. Somit wird insgesamt die gewünschte Progressivität entsprechend Kurve 71 in Fig. 2 erreicht.

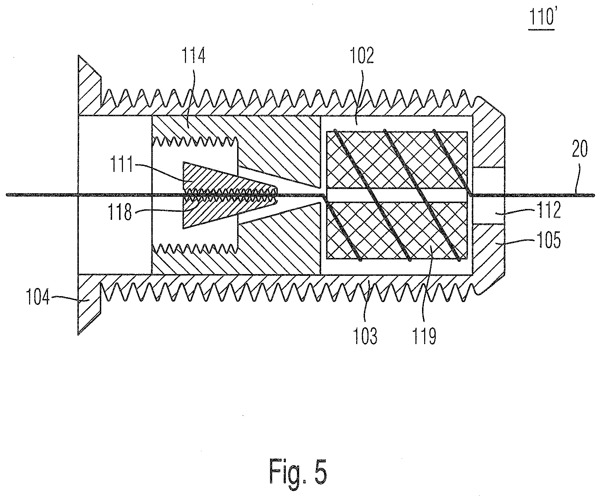

Fig. 5 zeigt ein Verankerungselement 110', das bezüglich Außenkörper 103 und Hülse 114 entsprechend dem Verankerungselement 110 aufgebaut ist. In den Innenraum 102 des Außenkörpers 103 ist wiederum ein Elastomerkörper 119 eingebracht. Der Elastomerkörper 119 weist eine zylindrische Form auf. Die Grundfläche des Elastomerkörpers ist bevorzugt rund, kann aber auch oval oder eckig ausgebildet sein. Das Verbindungselement 20 ist nun schraubenförmig bzw. spiralförmig um den Umfang des Elastomerkörpers 119 geführt und verlässt über die Öffnung 112 am Boden 105 des Außenkörpers 103 das Verankerungselement 110'.

Wirkt nun eine Zugbelastung auf das Verbindungselement 20, wird diese Zugkraft durch das Verbindungselement in radialer Richtung auf dem Elastomerkörper 119 übertragen. Bei einsetzender Zugbelastung lässt sich der Elastomerkörper 119 komprimieren und erreicht bei anhaltender Zugbelastung sein minimales Volumen und ist im Weiteren inkompressibel. Eine Kompression des Elastomerkörpers 119 in axialer Richtung erfolgt lediglich in einem geringem Maße, da der Großteil der Zugkraft zur Kompression des Elastomerkörpers 119 in radialer Richtung abgebaut wird.

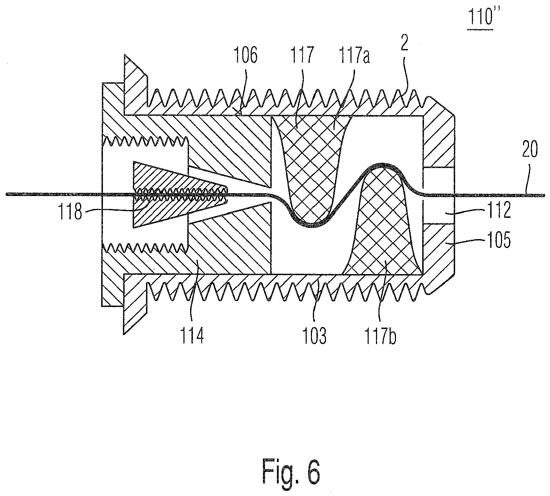

Um die zusätzliche axiale Kompression durch die Hülse 114 zu eliminieren, kann die Hülse 114 unbeweglich, wie .B. in Fig. 6 gezeigt, am Außenkörper fixiert sein. Dies führt zu einem weniger steilen Anstieg der Federkennlinie.

Um ein Verrutschen des Verbindungselements 20 am Umfang des Elastomerkörpers 119 zu verhindern, können Nute, nicht dargestellt, umfänglich am Elastomerkörper eingebracht werden, in denen das Verbindungselement 20 eingelegt und geführt wird.

Fig. 6 zeigt ein weiteres Ausführungsbeispiel der Dämpfungsvorrichtung durch einen Elastomerkörper 117, der eine Mehrzahl von zungenförmigen Ausbuchtungen 117a, 117b ausformt. Diese Ausbuchtungen 117a, 117b sind in axialer Richtung versetzt an der Innenseite 106 des Außenkörpers 103 angeordnet und nach radial innen ausgeformt. Sie erstrecken sich radial über die Achse des Verankerungselements 110' hinaus und sind in axialer Richtung voneinander beabstandet. Das Verbindungselement 20 wird vom Befestigungselement 118 gehalten und weiter um die zungenförmigen Ausbuchtungen 117a, 117b zur Öffnung 112 geführt.

Bei einer beginnenden Zugbelastung werden die Ausbuchtungen 117a, 117b in axiale Richtung zum Boden 105 des Außenkörpers 103 hin verformt. Dies entspricht wiederum dem flachen Bereich der Dehnungskennlinie 71 in Fig. 2. Bei zunehmender Zugbelastung wird das Verbindungselement 20 zwischen den nun aneinander liegenden Ausbuchtungen 117a, 117b verklemmt. Sind die Ausbuchtungen 117a, 117b bis maximal zur Anlage an den Boden 105 des Außenkörpers 103 hin verformt, ist keine weitere Kompression mehr möglich. Dies entspricht dem progressiven Verlauf der Dehnungskennlinie 71 in Fig. 2.

Der Anstieg und das Einsetzen des progressiven Bereichs der Dehnungskennlinie kann durch die Wahl des Materials für den Elastomerkörper 115, 117, 119 variiert werden. Geeignete Materialien für den Elastomerkörper 115, 117, 119 sind Polyethylen, Polyester, Polyurethan oder Silikon oder eine Mischung aus den genannten Materialien. Des Weiteren wird die Dehnungskennlinie durch das freie Volumen im Innenraum 102 des Außenkörpers 103 sowie die Form, den Durchmesser und die Länge des Elastomerkörpers 115, 117, 118 bestimmt.

Alle beschriebenen und/oder bezeichneten Merkmale können im Rahmen der Erfindung vorteilhaft miteinander kombiniert werden. Die Erfindung ist nicht auf die beschriebenen Ausführungsbeispiele beschränkt.

## Patentansprüche

1. System für eine kontrollierte Beanspruchung eines rekonstruierten oder renaturierten Bandes eines menschlichen oder tierischen Körpers, umfassend:
ein Verankerungselement (10) zur Implantation in einen ersten Knochen (41),
ein Verbindungselement (20),
ein Halteelement (30) zum Halten des Verbindungselements (20) an einem zweiten Knochen (51), und
einen Elastomerkörper (115, 117, 119) in einem Innenraum (102) eines Außenkörpers (103) des Verankerungselements (10),
wobei das Zusammenwirken des Elastomerkörpers (115, 117, 119) mit dem Verankerungselement (10) und dem Verbindungselement (20) zu einer Elastizität des Systems ähnlich dem Elastizitätsverlauf (71) eines natürlichen Bandes führt mit einem ersten Bereich eines flachen Verlaufs einer Dehnungskennlinie und einem zweiten Bereich eines progressiven Verlaufs der Dehnungskennlinie,
wobei der Elastomerkörper (115, 117, 119) ohne Zugbelastung des Verbindungselementes (20) den Innenraum (102) nicht komplett ausfüllt, so dass ein Volumen frei bleibt,
wobei bei zunehmender Zugbelastung am Verbindungselement (20) der Elastomerkörper (115, 117, 119) komprimiert wird und das freie Volumen im Innenraum (102) kontinuierlich ausfüllt, was zu dem flachen Verlauf der Dehnungskennlinie führt, und
wobei sobald der Elastomerkörper (115, 117, 119) das freie Volumen im wesentlichen ausfüllt und bei weiter zunehmender Zugbelastung inkompressibel ist, der progressive Verlauf der Dehnungskennlinie des Systems einsetzt.

2. System nach Anspruch 1,
**dadurch gekennzeichnet,**
**dass** der Elastomerkörper (115, 117, 119, 125) aus Polyethylen und/oder Polyester und/oder Polyurethan und/oder Silikon ist.

3. System nach Anspruch 1 oder 2,
**dadurch gekennzeichnet,**
**dass** der Elastomerkörper (115, 117, 119) im Innenraum (102) des Außenkörpers (103) des Verankerungselements (10) zwischen einer Anlagefläche (101) am Boden (105) des Außenkörpers (103) und einer Hülse (114), an der das Verbindungselement (20) fixiert ist, angeordnet ist.

4. System nach Anspruch 3,
**dadurch gekennzeichnet,**
**dass** die Hülse (114) beweglich im Außenkörper (103) gelagert ist.

5. System nach Anspruch 3,
**dadurch gekennzeichnet,**
**dass** die Hülse (114) unbeweglich am Außenkörper (103) fixiert ist.

6. System nach Anspruch 4,
**dadurch gekennzeichnet,**
**dass** das Verbindungselement (20) in einer axialen Aussparung (116) im Elastomerkörper (115) angeordnet ist.

7. System nach Anspruch 4 oder 5,
**dadurch gekennzeichnet,**
**dass** das Verbindungselement (20) spiralförmig um den Umfang des Elastomerkörpers (119) geführt ist.

8. System nach Anspruch 4, 5 oder 7,
**dadurch gekennzeichnet,**
**dass** der Elastomerkörper (119) umfänglich Nuten, die nicht parallel zur Längsachse des Elastomerkörpers (119) verlaufen, aufweist und das Verbindungselement (20) in diesen Nuten geführt ist.

9. System nach einem der Ansprüche 1 bis 8,
**dadurch gekennzeichnet,**
**dass** der Elastomerkörper (115, 117, 119, 125) eine zylindrische Form aufweist.

10. System nach Anspruch 4 oder 5,
**dadurch gekennzeichnet,**
**dass** der Elastomerkörper (117) als eine Mehrzahl von zungenförmigen Ausbuchtungen (117a, 117b) ausgeformt ist, wobei die Ausbuchtungen (117a, 117b) in axialer Richtung im Wechsel an gegenüberliegenden Seiten der Innenseite (106) des Außenkörpers (103) des Verankerungselements (110") nach innen gerichtet und in radialer Richtung überlappend angebracht sind und ein Zwischenraum zwischen den Ausbuchtungen (117a, 117b) ausgebildet ist, in dem das Verbindungselement (20) geführt ist.

## Claims

1. System for controlled stressing of a reconstructed or renatured ligament of a human or animal body, comprising:
an anchoring element (10) for implantation in a first bone (41),
a connecting element (20),
a holding element (30) for holding the connecting element (20) on a second bone (51), and
an elastomer body (115, 117, 119) in an internal space (102) of an external body (103) of the anchoring element (10),
wherein the interaction of the elastomer body (115, 117, 119) with the anchoring element (10) and the connecting element (20) leads to the system having an elasticity similar to the elasticity curve (71) of a natural ligament with a first region in which the course of an extension characteristic is flat and a second region in which the course of the extension characteristic is progressive,
wherein without tensile loading of the connecting element (20) the elastomer body (115, 117, 119) does not fill the internal space (102) completely so that a volume remains free,
wherein with increasing tensile loading at the connecting element (20) the elastomer body (115, 117, 119) is compressed and continuously fills the free volume in the internal space (102), leading to the flat course of the extension characteristic, and
wherein as soon as the elastomer body (115, 117, 119) essentially fills the free volume and is incompressible with further increasing tensile loading, the progressive course of the extension characteristic of the system starts.

2. System according to claim 1,
**characterised in that**
the elastomer body (115, 117, 119, 125) is made of polyethylene and/or polyester and/or polyurethane and/or silicone.

3. System according to claim 1 or 2,
**characterised in that**
the elastomer body (115, 117, 119) is arranged in the internal space (102) of the external body (103) of the anchoring element (10) between a bearing face (110) at the bottom (105) of the external body (103) and a sleeve (114) to which the connecting element (20) is fixed.

4. System according to claim 3,
**characterised in that**
the sleeve (114) is mounted so as to be able to move in the external body (103).

5. System according to claim 3,
**characterised in that**
the sleeve (114) is fixed immovably to the external body (103).

6. System according to claim 4,
**characterised in that**
the connecting element (20) is arranged in an axial recess (116) in the elastomer body (115).

7. System according to claim 4 or 5,
**characterised in that**
the connecting element (20) is guided in the form of a helix around the circumference of the elastomer body (119).

8. System according to claim 4, 5 or 7,
**characterised in that**
the elastomer body (119) has on its circumference grooves which do not run parallel to the longitudinal axis of the elastomer body (119) and the connecting element (20) is guided in these grooves.

9. System according to one of claims 1 to 8,
**characterised in that**
the elastomer body (115, 117, 119, 125) has a cylindrical shape.

10. System according to claim 4 or 5,
**characterised in that**
the elastomer body (117) is formed of a plurality of tongue-shaped protrusions (117a, 117b), wherein in the axial direction the protrusions (117a, 117b) are directed inwards in alternation on opposite sides of the inside (106) of the external body (103) of the anchoring element (110") and in the radial direction they are attached overlapping and an intermediate space is formed between the protrusions (117a, 117b) in which the connecting element (20) is guided.

## Revendications

1. Système destiné à une sollicitation contrôlée d'un ligament reconstruit ou renaturé d'un corps humain ou animal, comprenant :
un élément d'ancrage (10) destiné à être implanté dans un premier os (41),
un élément de liaison (20)
un élément de retenue (30) pour maintenir l'élément de liaison (20) sur un deuxième os (51), et
un corps en élastomère (115, 117, 119) dans une chambre intérieure (102) d'un corps extérieur (103) de l'élément d'ancrage (10),
système
dans lequel l'interaction du corps en élastomère (115, 117, 119) avec l'élément d'ancrage (10) et l'élément de liaison (20) conduit à une élasticité du système similaire à la loi de variation de l'élasticité (71) d'un ligament naturel, avec une première zone de variation d'allure plate d'une courbe caractéristique d'allongement et une deuxième zone de variation d'allure progressive de la courbe caractéristique d'allongement,
dans lequel le corps en élastomère (115, 117, 119), sans sollicitation de traction de l'élément de liaison (20), ne remplit pas complètement ladite chambre intérieure (102), de sorte qu'il reste un volume libre,
dans lequel lorsque la sollicitation de traction augmente sur l'élément de liaison (20), le corps en élastomère (115, 117, 119) est comprimé et remplit de manière continue le volume libre dans la chambre intérieure (102), ce qui conduit à la variation plate de la courbe caractéristique d'allongement, et
dans lequel dès que le corps en élastomère (115, 117, 119) remplit sensiblement le volume libre et est incompressible lorsque la sollicitation de traction continue à augmenter, débute la variation d'allure progressive de la courbe caractéristique d'allongement du système.

2. Système selon la revendication 1,
**caractérisé**
**en ce que** le corps en élastomère (115, 117, 119, 125) est réalisé en polyéthylène et/ou en polyester et/ou polyuréthanne et/ou en silicone.

3. Système selon la revendication 1 ou la revendication 2,
**caractérisé**
**en ce que** le corps en élastomère (115, 117, 119) est agencé dans la chambre intérieure (102) du corps extérieur (103) de l'élément d'ancrage (10), entre une surface d'appui (101) sur le fond (105) du corps extérieur (103), et une douille (114) sur laquelle est fixé l'élément de liaison (20).

4. Système selon la revendication 3,
**caractérisé**
**en ce que** la douille (114) est montée mobile dans le corps extérieur (103).

5. Système selon la revendication 3,
**caractérisé**
**en ce que** la douille (114) est fixée dans le corps extérieur (103) de manière à être non mobile.

6. Système selon la revendication 4,
**caractérisé**
**en ce que** l'élément de liaison (20) est agencé dans un évidement axial (116) dans le corps en élastomère (115).

7. Système selon la revendication 4 ou la revendication 5,
**caractérisé**
**en ce que** l'élément de liaison (20) est guidé en forme de spirale autour de la périphérie du corps en élastomère (119).

8. Système selon la revendication 4, 5 ou la revendication 7,
**caractérisé**
**en ce que** le corps en élastomère (119) comporte, en périphérie, des rainures qui ne s'étendent pas parallèlement à l'axe longitudinal du corps en élastomère (119), et l'élément de liaison (20) est guidé dans ces rainures.

9. Système selon l'une des revendications 1 à 8,
**caractérisé**
**en ce que** le corps en élastomère (115, 117, 119, 125) présente une forme cylindrique.

10. Système selon la revendication 4 ou la revendication 5,
**caractérisé**
**en ce que** le corps en élastomère (117) est formé sous la forme d'une pluralité de proéminences (117a, 117b) en forme de languette, les proéminences (117a, 117b) étant placées de manière alternée en direction axiale, sur des côtés mutuellement opposés du côté intérieur (106) du corps extérieur (103) de l'élément d'ancrage (110"), en étant dirigées vers l'intérieur et en se chevauchant dans la direction radiale, et un espace intermédiaire dans lequel est guidé l'élément de liaison (20), étant formé entre les proéminences (117a, 117b).
